# EUROPÄISCHE PATENTSCHRIFT

(11) **EP 1 858 858 B1**
(45) Veröffentlichungstag und Bekanntmachung des Hinweises auf die Patenterteilung: **29.04.2015**
(21) Anmeldenummer: 06723163.9
(22) Anmeldetag: 01.03.2006
(51) Int. Cl.: C07D 231/16, C07D 413/12

(54) **VERFAHREN ZUM HERSTELLEN VON ALKYLANILIDEN**
METHOD FOR PRODUCING ALKYL ANILIDES
PROCEDE DE PRODUCTION D'ALKYLANILIDES

(30) Priorität: 02.03.2005 DE 102005009457
(43) Veröffentlichungstag der Anmeldung: 28.11.2007
(73) Patentinhaber: Bayer Intellectual Property GmbH, 40789 Monheim am Rhein (DE)
(72) Erfinder: STRAUB, Alexander, 42117 Wuppertal (DE)
(74) Vertreter: BIP Patents
(86) Internationale Anmeldenummer: PCT/EP2006/001871
(87) Internationale Veröffentlichungsnummer: WO 2006/092291

(56) Entgegenhaltungen:
- EP-A- 0 824 099
- WO-A-03/010149
- WO-A-2004/103953
- WO-A-2004/103975
- WO-A-2005/075452
- TARZIA, GIORGIO ET AL: "Synthesis and structure-activity relationships of a series of pyrrole cannabinoid receptor agonists" BIOORGANIC & MEDICINAL CHEMISTRY , 11(18), 3965-3973 CODEN: BMECEP; ISSN: 0968-0896, 2003, XP002394111
- HAHN H-G ET AL: "SYNTHESIS OF TRIFLUOROMETHYLATED DIHYDRO-1,4-OXATHIN-3-CARBOXANILIDES THROUGH POLYMER-BOUND ACTIVATED ESTER" HETEROCYCLES, ELSEVIER SCIENCE PUBLISHERS B.V. AMSTERDAM, NL, Bd. 48, Nr. 11, 1998, Seiten 2253-2261, XP009038022 ISSN: 0385-5414
- DATABASE CA [Online] CHEMICAL ABSTRACTS SERVICE, COLUMBUS, OHIO, US; HAHN, HOH-GYU ET AL: "Anchimeric assistance in the rearrangement of dichloro-3-methyl-1,4- oxathianes to 2-chloromethyl dihydro-1,4-oxathiins" XP002394122 gefunden im STN Database accession no. 1998:699952 & BULLETIN OF THE KOREAN CHEMICAL SOCIETY , 19(10), 1109-1112 CODEN: BKCSDE; ISSN: 0253-2964, 1998,
- DATABASE CA [Online] CHEMICAL ABSTRACTS SERVICE, COLUMBUS, OHIO, US; KOVENDI, ALEXANDER ET AL: "New synthesis for quinazoline N3-oxides and 1,2-dihydroquinazoline N3-oxides" XP002394123 gefunden im STN Database accession no. 1965:90918 & CHEMISCHE BERICHTE , 98(4), 1049-59 CODEN: CHBEAM; ISSN: 0009-2940, 1965,
- BOGERT, MARSTON TAYLOR ET AL: "Quinazolines. XXXVIII. Synthesis of some new analogs of cinchophen and intermediate products" JOURNAL OF THE AMERICAN CHEMICAL SOCIETY , 49, 2650-4 CODEN: JACSAT; ISSN: 0002-7863, 1927, XP002394112
- DATABASE REGISTRY [Online] Chemical Abstracts Service, Columbus, Ohio, US; Chemical Library; Supplier: Enamine 9. Dezember 2002 (2002-12-09), XP002394124 gefunden im STN Database accession no. RN - 795292-48-7
- DATABASE REGISTRY [Online] Chemical Abstracts Service, Columbus, Ohio, US; Chemical Library; Supplier: Enamine 30. November 2004 (2004-11-30), XP002394125 gefunden im STN Database accession no. RN - 790679-71-9
- DATABASE REGISTRY [Online] Chemical Abstracts Service, Columbus, Ohio, US; Chemical LIbrary; Supplier: Scientific Exchange 10. November 2004 (2004-11-10), XP002394126 gefunden im STN Database accession no. RN - 777875-56-6
- DATABASE REGISTRY [Online] Chemical Abstracts Service, Columbus, Ohio, US; Chemical Library; Supplier: ChemBridge Corp. 4. Juni 2002 (2002-06-04), XP002394127 gefunden im STN Database accession no. RN - 425399-14-0
- DATABASE REGISTRY [Online] Chemical Abstracts Service, Columbus, Ohio, US; CAS Client Services 2. Oktober 1997 (1997-10-02), XP002394128 gefunden im STN Database accession no. RN - 194783-80-7
- DIXON W J ET AL: "Kinetics and mechanism of the addition of water and ring-opening of 2-methyl- and 2-aryl-4H-3,1-benzoxazines to 2-aminobenzyl esters in the acidic pH range; change in rate-limiting step with buffer concentration and evidence for a tetrahedral carbonyl addition intermediate" JOURNAL OF THE CHEMICAL SOCIETY, PERKIN TRANSACTIONS 2, CHEMICAL SOCIETY. LETCHWORTH, GB, Bd. 8, 1997, Seiten 1503-1510, XP002323920 ISSN: 1472-779X
- HARI ET AL: "Efficient synthesis of o-alkynyl-N-pivaloylanilines from o-acyl-N-pivaloylanilines and lithium trimethylsilyldiazomethane" TETRAHEDRON LETTERS, ELSEVIER, AMSTERDAM, NL, Bd. 47, Nr. 7, 13. Februar 2006 (2006-02-13), Seiten 1137-1139, XP005247514 ISSN: 0040-4039
- DATABASE REGISTRY [Online] Chemical Abstracts Service, Columbus, Ohio, US; Chemical Library; Supplier: Enamine 4. Juli 2005 (2005-07-04), XP002394129 gefunden im STN Database accession no. RN - 853689-32-4
- DATABASE REGISTRY [Online] Chemical Abstracts Service, Columbus, Ohio, US; Chemical Library; Supplier: Enamine 11. April 2005 (2005-04-11), XP002394130 gefunden im STN Database accession no. RN - 848249-78-5

## Beschreibung

Die vorliegende Erfindung betrifft Benzoxazin-Derivate.

Es ist bereits bekannt, dass man Alkylanilide durch Umsetzung des gewünschten Säurechlorids mit dem entsprechenden Alkylanilin-Derivat erhält (vgl. EP-A 0 824 099, WO 03/010149).

Weiterhin ist bekannt, dass man Alkylanilide erhält, indem man die entsprechenden Alkenyl- oder Alkinylanilide hydriert, wobei man die Alkenylanilide aus Acylaniliden durch Umsetzung mit einer Phosphorsäureverbindung in einer Wittig-artigen Reaktion erhalten kann (vgl WO 03/010149).

Gegenstand der vorliegenden Erfindung sind Benzoxazine der Formel (VIIa) 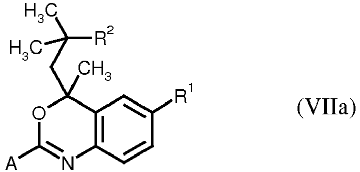 in welcher
- R¹: für Wasserstoff oder Fluor steht,
- R²: für Wasserstoff, Halogen, C₁-C₄-Alkyl oder C₁-C₄-Halogenalkyl mit 1 bis 9 Fluor-, Chlor- und/oder Bromatomen steht,
- A: für den Rest der Formel (A1) 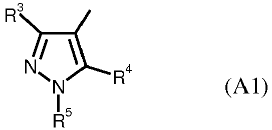 steht, in welcher
- R³: für Wasserstoff, Cyano, Halogen, Nitro, C₁-C₄-Alkyl C₁-C₄-Alkoxy, C₁-C₄-Alkylthio, C₃-C₆-Cycloalkyl, C₁-C₄-Halogenalkyl C₁-C₄-Halogena]koxy oder C₁-C₄-Halogenalkylthio mit jeweils 1 bis 5 Halogenatomen, Aminocarbonyl oder Aminocarbonyl-C₁-C₄-alkyl steht,
- R⁴: für Wasserstoff, Halogen, Cyano, C₁-C₄-Alkyl C₁-C₄-Alkoxy oder C₁-C₄-Alkylthio steht,
- R⁵: für Wasserstoff, C₁-C₄-Alkyl Hydroxy-C₁-C₄-alkyl, C₂-C₆-Alkenyl, C₃-C₆-Cycloalkyl, C₁-C₄-Alkylthio-C₁-C₄-alkyl, C₁-C₄-Alkoxy-Cᵢ-C₄-alkyl C₁-C₄-Halogenalkyl, C₁-C₄-Halogenalkylthio-C₁-C₄-alkyl C₁-C₄-Halogenalkoxy-C₁-C₄-alkyl mit jeweils 1 bis 5 Halogenatomen, oder Phenyl steht,
oder
- A: für den Rest der Formel (A2) 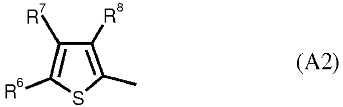 steht, in welcher

- R⁶ und R⁷: unabhängig voneinander für Wasserstoff, Halogen, C₁-C₄-Alkyl oder C₁-C₄-Halogenalkyl mit 1 bis 5 Halogenatomen steht,
- R⁸: für Halogen, Cyano oder C₁-C₄-Alkyl, oder C₁-C₄-Halogenalkyl oder C₁-C₄-Halogenalkoxy mit jeweils 1 bis 5 Halogenatomen steht,
oder
- A: für den Rest der Formel (A3) 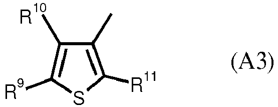 steht, in welcher
- R⁹ und R¹⁰: unabhängig voneinander für Wasserstoff, Halogen, C₁-C₄-Alkyl oder C₁-C₄-Halogenalkyl mit 1 bis 5 Halogenatomen steht,
- R¹¹: für Wasserstoff, Halogen, C₁-C₄-Alkyl oder C₁-C₄-Halogenalkyl mit 1 bis 5 Halogenatomen steht,
oder
- A: für den Rest der Formel (A4) 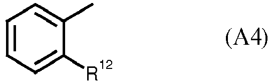 steht, in welcher
- R¹²: für Wasserstoff, Halogen, Hydroxy, Cyano, C₁-C₆-Alkyl, C₁-C₄-Halogenalkyl, C₁-C₄-Halogenalkoxy oder C₁-C₄-Halogenalkylthio mit jeweils 1 bis 5 Halogenatomen steht,
oder
- A: für den Rest der Formel (A5) 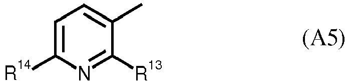 steht, in welcher
- R¹³: für Halogen, Hydroxy, Cyano, C₁-C₄-Alkyl, C₁-C₄-Alkoxy, C₁-C₄-Alkylthio, C₁-C₄-Halogenalkyl, C₁-C₄-Halogenalkylthio oder C₁-C₄-Halogenalkoxy mit jeweils 1 bis 5 Halogenatomen steht,
- R¹⁴: für Wasserstoff, Halogen, Cyano, C₁-C₄-Alkyl, C₁-C₄-Alkoxy, C₁-C₄-Alkylthio, C₁-C₄-Halogenalkyl, C₁-C₄-Halogenalkoxy mit jeweils 1 bis 5 Halogenatomen, C₁-C₄-Alkylsulphinyl oder C₁-C₄-Alkylsulphonyl steht,
oder
- A: für den Rest der Formel (A6) 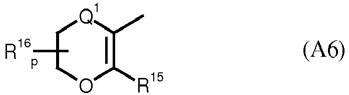 steht, in welcher
- R¹⁵: für C₁-C₄-Alkyl oder C₁-C₄-Halogenalkyl mit 1 bis 5 Halogenatomen steht,
- R¹⁶: für C₁-C₄-Alkyl steht,
- Q¹: für S (Schwefel), O (Sauerstoff), SO, SO₂ oder CH₂ steht,
- p: für 0, 1 oder 2, wobei R¹⁶ für identische oder verschiedene Reste steht, wenn p für 2 steht,
oder
- A: für den Rest der Formel (A7) 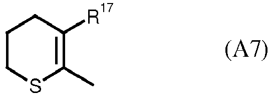 steht, in welcher
- R¹⁷: für C₁-C₄-Alkyl oder C₁-C₄-Halogenalkyl mit 1 bis 5 Halogenatomen steht,
oder
- A: für den Rest der Formel (A8) 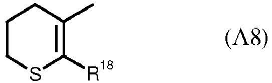 steht, in welcher
- R¹⁸: für C₁-C₄-Alkyl oder C₁-C₄-Halogenalkyl mit 1 bis 5 Halogenatomen steht,
oder
- A: für den Rest der Formel (A9) 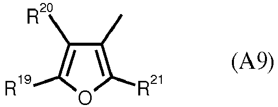 steht, in welcher
- R¹⁹ und R²⁰: unabhängig voneinander für Wasserstoff, Halogen, Amino, C₁-C₄-Alkyl oder C₁-C₄-Halogenalkyl mit 1 bis 5 Halogenatomen stehen,
- R²¹: für Wasserstoff, Halogen, C₁-C₄-Alkyl oder C₁-C₄-Halogenalkyl mit 1 bis 5 Halogenatomen steht,
oder
- A: für den Rest der Formel (A10) 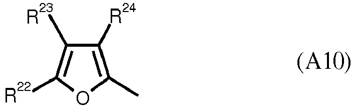 steht, in welcher
- R²² und R²³: unabhängig voneinander für Wasserstoff, Halogen, Amino, Nitro, C₁-C₄-Alkyl oder C₁-C₄-Halogenalkyl having 1 bis 5 Halogenatomen stehen,
- R²⁴: für Wasserstoff, Halogen, C₁-C₄-Alkyl oder C₁-C₄-Halogenalkyl mit 1 bis 5 Halogenatomen steht,
oder
- A: für den Rest der Formel (A 11) 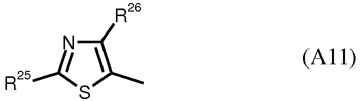 steht, in welcher
- R²⁵: für Wasserstoff, Halogen, Amino, C₁-C₄-Alkylamino, Di-(C₁-C₄-alkyl)amino, Cyano, C₁-C₄-Alkyl oder C₁-C₄-Halogenalkyl mit 1 bis 5 Halogenatomen steht,
- R²⁶: für Halogen, C₁-C₄-Alkyl oder C₁-C₄-Halogenalkyl mit 1 bis 5 Halogenatomen steht,
oder
- A: für den Rest der Formel (A 12) 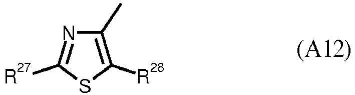 steht, in welcher
- R²⁷: für Wasserstoff, Halogen, Amino, C₁-C₄-Alkylamino, Di-(C₁-C₄-alkyl)amino, Cyano, C₁-C₄-Alkyl oder C₁-C₄-Halogenalkyl mit 1 bis 5 Halogenatomen steht,
- R²⁸: für Halogen, C₁-C₄-Alkyl oder C₁-C₄-Halogenalkyl mit 1 bis 5 Halogenatomen steht,
oder
- A: für den Rest der Formel (A13) 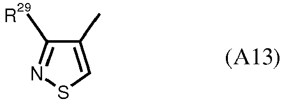 steht, in welcher
- R²⁹: für Halogen, C₁-C₄-Alkyl oder C₁-C₄-Halogenalkyl mit 1 bis 5 Halogenatomen steht,
oder
- A: für den Rest der Formel (A 14) 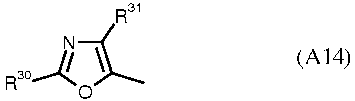 steht, in welcher
- R³⁰: für Wasserstoff oder C₁-C₄-Alkyl steht,
- R³¹: für Halogen oder C₁-C₄-Alkyl steht,
oder
- A: für den Rest der Formel (A15) 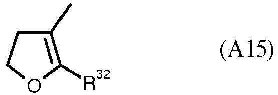 steht, in welcher
- R³²: für C₁-C₄-Alkyl oder C₁-C₄-Halogenalkyl mit 1 bis 5 Halogenatomen steht,
oder
- A: für den Rest der Formel (A16) 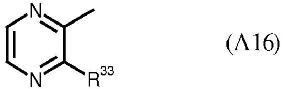 steht, in welcher
- R³³: für Wasserstoff, Halogen, C₁-C₄-Alkyl oder C₁-C₄-Halogenalkyl mit 1 bis 5 Halogenatomen steht,
oder
- A: für den Rest der Formel (A 17) 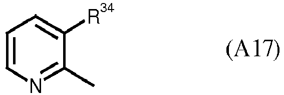 steht, in welcher
- R³⁴: für Halogen, Hydroxy, C₁-C₄-Alkyl, C₁-C₄-Alkoxy, C₁-C₄-Alkylthio, C₁-C₄-Halogenalkyl, C₁-C₄-Halogenalkylthio oder C₁-C₄-Halogenalkoxy mit jeweils 1 bis 5 Halogenatomen steht,
oder
- A: für den Rest der Formel (A18) 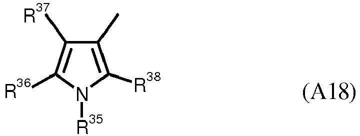 steht, in welcher
- R³⁵: für Wasserstoff, Cyano, C₁-C₄-Alkyl, C₁-C₄-Halogenalkyl mit 1 bis 5 Halogenatomen, C₁-C₄-Alkoxy-C₁-C₄-alkyl, Hydroxy-C₁-C₄-alkyl, C₁-C₄-Alkylsulfonyl, Di(C₁-C₄-alkyl)aminosulfonyl, C₁-C₆-Alkylcarbonyl oder jeweils gegebenenfalls substituiertes Phenylsulfonyl oder Benzoyl steht,
- R³⁶: für Wasserstoff, Halogen, C₁-C₄-Alkyl oder C₁-C₄-Halogenalkyl mit 1 bis 5 Halogenatomen steht,
- R³⁷: für Wasserstoff, Halogen, Cyano, C₁-C₄-Alkyl oder C₁-C₄-Halogenalkyl mit 1 bis 5 Halogenatomen steht,
- R³⁸: für Wasserstoff, Halogen, C₁-C₄-Alkyl oder C₁-C₄-Halogenalkyl mit 1 bis 5 Halogenatomen steht,
oder
- A: für den Rest der Formel (A19) 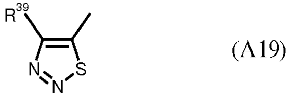 steht, in welcher
- R³⁹: für C₁-C₄-Alkyl steht.

### Verfahren zur Herstellung

Verfahren zur Herstellung von Benzoxazinen der Formel (VIIa),
dadurch gekennzeichnet, dass man
Alkylanilide der Formel (I) 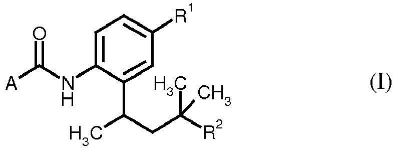 in welchen R¹, R² und A die oben angegebenen Bedeutungen haben,
a) in einem ersten Schritt mit Carbonsäurehalogenide der Formel (II) 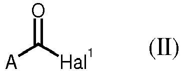 in welcher
   - A: die oben angegebenen Bedeutungen hat und
   - Hal¹: für Fluor, Chlor oder Brom steht,
   mit Acetophenon-Derivaten der Formel (III) oder deren Salze 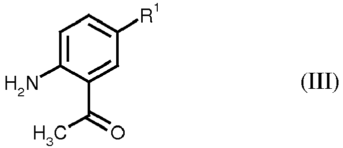 in welcher R¹ die oben angegebenen Bedeutungen hat,
   gegebenenfalls in Gegenwart eines Säurebindemittels und gegebenenfalls in Gegenwart eines Verdünnungsmittels umsetzt,
   und
b) die so erhaltenen Acetylanilide der Formel (IV) 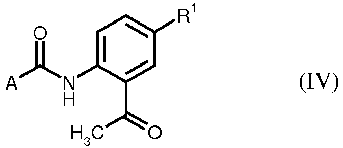 in welcher A und R¹ die oben angegebenen Bedeutungen haben,
   in einem zweiten Schritt mit einem Grignard-Reagenz der Formel (V) 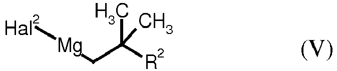 in welcher
   - R²: die oben angegebenen Bedeutungen hat und
   - Hal²: für Chlor, Brom oder Iod steht,
   in Gegenwart eines Verdünnungsmittels umsetzt,
   und
c) die so erhaltenen Hydroxyalkylcarboxanilide der Formel (VI) 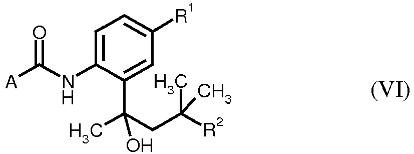 in welcher R¹, R² und A die oben angegebenen Bedeutungen haben,
   in Gegenwart einer Säure und in Gegenwart eines Verdünnungsmittels umsetzt, und die Benzoxazine der Formel (VIIa) und Alkenylanilide der Formeln (VIIb) und (VIIc) 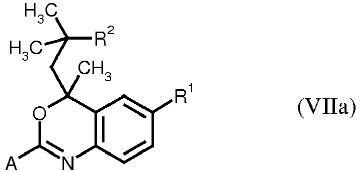 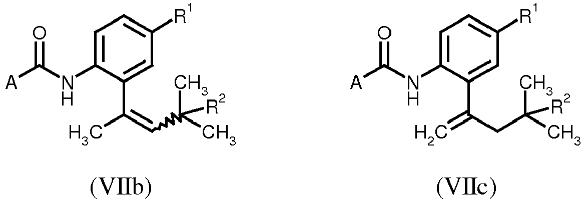 erhält.

Überraschenderweise erhält man bei der dritten Stufe (Abspaltung von Wasser) bei kurzen Reaktionszeiten bevorzugt das Oxazin der allgemeinen Formel (VIIa), während bei längeren Reaktionszeiten und mit anderen wasserabspaltenden Reagenzien die bevorzugte Bildung der Alkenylanilide (VIIb) und (VIIc) beobachtet wird. Diese können wiederum unter milderen Bedingungen hydriert werden.

Verwendet man beispielsweise 1,3-Dimethyl-5-fluor-4-pyrazolcarbonsäurechlorid und 2-Amino-acetophenon als Ausgangsstoffe kann das erfindungsgemäße Verfahren durch das folgende Formelschema veranschaulicht werden:

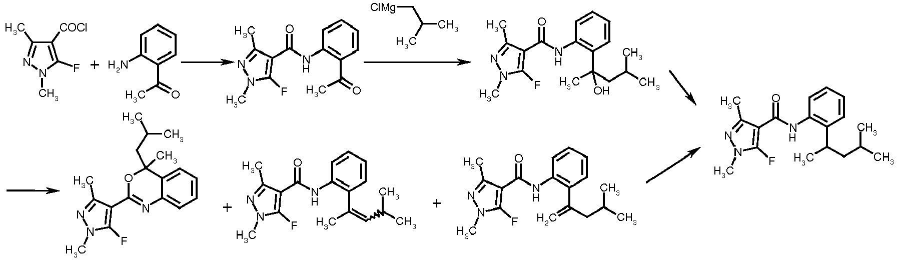

Die bei der Durchführung des erfindungsgemäßen Verfahrens als Ausgangsstoffe verwendeten Carbonsäurehalogenide sind durch die Formel (II) allgemein definiert. Hal¹ steht bevorzugt für Fluor oder Chlor.

Der Substituent A hat die oben angegebenen Bedeutungen A1 bis A19.
- A: steht bevorzugt für einen der Reste A1, A2, A3, A4, A5, A6, A9, A10, A11, A12, A16, A17 oder A18.
- A: steht besonders bevorzugt für einen der Reste A1, A2, A3, A4, A5, A6, A9, A11, A16, A17, A18.
- A: ganz besonders bevorzugt für den Rest A1.
- A: außerdem ganz besonders bevorzugt für den Rest A2.
- A: außerdem ganz besonders bevorzugt für den Rest A3.
- A: außerdem ganz besonders bevorzugt für den Rest A4.
- A: außerdem ganz besonders bevorzugt für den Rest A5.
- A: außerdem ganz besonders bevorzugt für den Rest A6.
- A: außerdem ganz besonders bevorzugt für den Rest A9.
- A: außerdem ganz besonders bevorzugt für den Rest A11.
- A: außerdem ganz besonders bevorzugt für den Rest A16.
- A: außerdem ganz besonders bevorzugt für den Rest A17.
- A: außerdem ganz besonders bevorzugt für den Rest A18.

In den Resten A1 bis A19 haben die oben angegebenen Substituenten folgende bevorzugten Bedeutungen.
- R³: steht bevorzugt für Wasserstoff, Cyano, Fluor, Chlor, Brom, Iod, Methyl, Ethyl, iso-Propyl, Methoxy, Ethoxy, Methylthio, Ethylthio, Cyclopropyl, C₁-C₂-Halogenalkyl C₁-C₂-Halogenalkoxy mit jeweils 1 bis 5 Fluor, Chlor und/oder Bromatomen, Trifluormethylthio, Difluormethylthio, Aminocarbonyl, Aminocarbonylmethyl oder Aminocarbonylethyl
- R³: steht besonders bevorzugt für Wasserstoff, Fluor, Chlor, Brom, Iod, Methyl, Ethyl, iso-Propyl, Monofluormethyl, Monofluorethyl, Difluormethyl, Trifluormethyl, Difluorchlormethyl, Trichlormethyl Dichlormethyl, Cyclopropyl, Methoxy, Ethoxy, Trifluormethoxy, Trichlormethoxy, Methylthio, Ethylthio, Trifluormethylthio oder Difluormethylthio.
- R³: steht ganz besonders bevorzugt Wasserstoff, Fluor, Chlor, Brom, Iod, Methyl, iso-Propyl, Monofluormethyl, Monofluorethyl, Difluormethyl, Trifluormethyl, Difluorchlormethyl oder Trichlormethyl
- R³: steht insbesondere bevorzugt für Methyl, Difluormethyl, Trifluormethyl oder 1-Fluorethyl
- R⁴: steht bevorzugt für Wasserstoff, Fluor, Chlor, Brom, Iod, Methyl, Ethyl, Methoxy, Ethoxy, Methylthio oder Ethylthio.
- R⁴: steht besonders bevorzugt für Wasserstoff, Fluor, Chlor, Brom, Iod oder Methyl.
- R⁴: steht ganz besonders bevorzugt für Wasserstoff, Fluor, Chlor oder Methyl.
- R⁵: steht bevorzugt für Wasserstoff, Methyl, Ethyl, n-Propyl, iso-Propyl, C₁-C₂-Halogenalkyl mit 1 bis 5 Fluor, Chlor und/oder Bromatomen, Hydroxymethyl, Hydroxyethyl, Cyclopropyl, Cyclopentyl, Cyclohexyl oder Phenyl.
- R⁵: steht besonders bevorzugt für Wasserstoff, Methyl, Ethyl, iso-Propyl, Trifluormethyl, Difluormethyl, Hydroxymethyl, Hydroxyethyl oder Phenyl
- R⁵: steht ganz besonders bevorzugt für Wasserstoff, Methyl, Trifluormethyl oder Phenyl
- R⁵: steht insbesondere bevorzugt für Methyl.
- R⁶ und R⁷: stehen unabhängig voneinander bevorzugt für Wasserstoff, Fluor, Chlor, Brom, Methyl, Ethyl oder C₁-C₂-Halogenalkyl mit 1 bis 5 Fluor, Chlor und/oder Bromatomen.
- R⁶ und R⁷: stehen unabhängig voneinander besonders bevorzugt für Wasserstoff, Fluor, Chlor, Brom, Methyl, Ethyl, Difluormethyl Trifluormethyl Difluorchlormethyl oder Trichlormethyl.
- R⁶ und R⁷: stehen unabhängig voneinander ganz besonders bevorzugt für Wasserstoff, Fluor, Chlor, Brom, Methyl, Ethyl, Difluormethyl, Trifluormethyl oder Trichlormethyl
- R⁶ und R⁷: stehen insbesondere bevorzugt jeweils für Wasserstoff.
- R⁸: steht bevorzugt für Fluor, Chlor, Brom, Cyano, Methyl, Ethyl, C₁-C₂-Halogenalkyl oder C₁-C₂-Halogenalkoxy mit jeweils 1 bis 5 Fluor, Chlor und/oder Bromatomen.
- R⁸: steht besonders bevorzugt für Fluor, Chlor, Brom, Cyano, Methyl, Trifluormethyl, Trifluormethoxy, Difluormethoxy, Difluorchlormethoxy oder Trichlormethoxy.
- R⁸: steht ganz besonders bevorzugt für Fluor, Chlor, Brom, Iod, Methyl, Trifluormethyl oder Trifluormethoxy.
- R⁸: steht insbesondere bevorzugt für Methyl.
- R⁹ und R¹⁰: stehen unabhängig voneinander bevorzugt für Wasserstoff, Fluor, Chlor, Brom, Methyl, Ethyl oder C₁-C₂-Halogenalkyl mit 1 bis 5 Fluor, Chlor und/oder Bromatomen.
- R⁹ und R¹⁰: stehen unabhängig voneinander besonders bevorzugt für Wasserstoff, Fluor, Chlor, Brom, Methyl, Ethyl, Difluormethyl, Trifluormethyl, Difluorchlormethyl oder Trichlormethyl.
- R⁹ und R¹⁰: stehen unabhängig voneinander ganz besonders bevorzugt für Wasserstoff, Fluor, Chlor, Brom, Methyl, Ethyl, Difluormethyl, Trifluormethyl oder Trichlormethyl.
- R⁹ und R¹⁰: stehen insbesondere bevorzugt jeweils für Wasserstoff.
- R¹¹: steht bevorzugt für Wasserstoff, Fluor, Chlor, Brom, Methyl, Ethyl oder C₁-C₂-Halogenalkyl mit 1 bis 5 Fluor, Chlor und/oder Bromatomen.
- R¹¹: steht besonders bevorzugt für Wasserstoff, Fluor, Chlor, Brom, Iod, Methyl oder Trifluormethyl.
- R¹¹: steht ganz besonders bevorzugt für Methyl.
- R¹²: steht bevorzugt für Wasserstoff, Fluor, Chlor, Brom, Iod, Hydroxy, Cyano, C₁-C₄-Alkyl, C₁-C₂-Halogenalkyl, C₁-C₂-Halogenalkoxy oder C₁-C₂-Halogenalkylthio mit jeweils 1 bis 5 Fluor, Chlor und/oder Bromatomen.
- R¹²: steht besonders bevorzugt für Wasserstoff, Fluor, Chlor, Brom, Iod, Hydroxy, Cyano, Methyl, Ethyl, n-Propyl, iso-Propyl, n-Butyl, iso-Butyl, sec-Butyl, tert-Butyl, Difluormethyl, Trifluormethyl, Difluorchlormethyl, Trichlormethyl, Trifluormethoxy, Difluormethoxy, Difluorchlormethoxy, Trichlormethoxy, Trifluormethylthio, Difluormethylthio, Difluorchlormethylthio oder Trichlormethylthio.
- R¹²: steht ganz besonders bevorzugt für Wasserstoff, Fluor, Chlor, Brom, Iod, Methyl, Difluor-methyl, Trifluormethyl oder Trichlormethyl.
- R¹²: steht insbesondere bevorzugt für Iod, Methyl, Difluormethyl oder Trifluormethyl.
- R¹³: steht bevorzugt für Fluor, Chlor, Brom, Iod, Hydroxy, Cyano, C₁-C₄-Alkyl, Methoxy, Ethoxy, Methylthio, Ethylthio, Difluormethylthio, Trifluormethylthio, C₁-C₂-Halogenalkyl oder C₁-C₂-Halogenalkoxy mit jeweils 1 bis 5 Fluor, Chlor und/oder Bromatomen.
- R¹³: steht besonders bevorzugt für Fluor, Chlor, Brom, Iod, Hydroxy, Cyano, Methyl, Ethyl, n-Propyl, iso-Propyl, n-Butyl, iso-Butyl, sec-Butyl, tert-Butyl, Trifluormethyl, Difluormethyl, Difluorchlormethyl, Trichlormethyl, Methoxy, Ethoxy, Methylthio, Ethylthio, Difluormethylthio, Trifluormethylthio, Trifluormethoxy, Difluormethoxy, Difluorchlormethoxy oder Trichlormethoxy.
- R¹³: steht ganz besonders bevorzugt für Fluor, Chlor, Brom, Iod, Methyl, Trifluormethyl, Difluormethyl oder Trichlormethyl.
- R¹⁴: steht bevorzugt für Wasserstoff, Fluor, Chlor, Brom, Iod, Cyano, C₁-C₄-Alkyl, Methoxy, Ethoxy, Methylthio, Ethylthio, C₁-C₂-Halogenalkyl oder C₁-C₂-Halogenalkoxy mit jeweils 1 bis 5 Fluor, Chlor und/oder Bromatomen, C₁-C₂-Alkylsulphinyl oder C₁-C₂-Alkylsulphonyl.
- R¹⁴: steht besonders bevorzugt für Wasserstoff, Fluor, Chlor, Brom, Iod, Cyano, n-Propyl, iso-Propyl, n-Butyl, iso-Butyl, sec-Butyl, tert-Butyl, Trifluormethyl, Difluormethyl, Difluorchlormethyl, Trichlormethyl, Methoxy, Ethoxy, Methylthio, Ethylthio, Trifluormethoxy, Difluormethoxy, Difluorchlormethoxy, Trichlormethoxy, Methylsulphinyl oder Methylsulphonyl.
- R¹⁴: steht ganz besonders bevorzugt für Wasserstoff, Fluor, Chlor, Brom, Iod, n-Propyl, iso-Propyl, n-Butyl, iso-Butyl, sec-Butyl, tert-Butyl, Trifluormethyl, Difluormethyl, Trichlormethyl, Methylsulphinyl oder Methylsulphonyl.
- R¹⁴: steht insbesondere bevorzugt für Wasserstoff.
- R¹⁵: steht bevorzugt für Methyl, Ethyl oder C₁-C₂-Halogenalkyl mit 1 bis 5 Fluor, Chlor und/oder Bromatomen.
- R¹⁵: steht besonders bevorzugt für Methyl, Ethyl, Trifluormethyl, Difluormethyl, Difluorchlormethyl oder Trichlormethyl.
- R¹⁶: steht bevorzugt für Methyl oder Ethyl.
- R¹⁶: steht besonders bevorzugt für Methyl.
- Q¹: steht bevorzugt für S (Schwefel), SO₂ oder CH₂.
- Q¹: steht besonders bevorzugt für S (Schwefel) oder CH₂.
- Q¹: steht glanz besonders bevorzugt für S (Schwefel).
- p: steht bevorzugt für 0 oder 1.
- p: steht besonders bevorzugt für 0.
- R¹⁷: steht bevorzugt für Methyl, Ethyl oder C₁-C₂-Halogenalkyl mit 1 bis 5 Fluor, Chlor und/oder Bromatomen.
- R¹⁷: steht besonders bevorzugt für Methyl, Ethyl, Trifluormethyl Difluormethyl, Difluorchlormethyl oder Trichlormethyl.
- R¹⁷: steht ganz besonders bevorzugt für Methyl, Trifluormethyl, Difluormethyl oder Trichlormethyl.
- R¹⁸: steht bevorzugt für Methyl, Ethyl oder C₁-C₂-Halogenalkyl mit 1 bis 5 Fluor, Chlor und/oder Bromatomen.
- R¹⁸: steht besonders bevorzugt für Methyl, Ethyl, Trifluormethyl, Difluormethyl, Difluorchlormethyl oder Trichlormethyl.
- R¹⁸: steht ganz besonders bevorzugt für Methyl, Trifluormethyl, Difluormethyl oder Trichlor-methyl
- R¹⁹ und R²⁰: stehen unabhängig voneinander bevorzugt für Wasserstoff, Fluor, Chlor, Brom, Amino, Methyl, Ethyl oder C₁-C₂-Halogenalkyl mit 1 bis 5 Fluor, Chlor und/oder Bromatomen.
- R¹⁹ und R²⁰: stehen unabhängig voneinander besonders bevorzugt für Wasserstoff, Fluor, Chlor, Brom, Methyl, Ethyl, Trifluormethyl, Difluormethyl, Difluorchlormethyl oder Trichlormethyl.
- R¹⁹ und R²⁰: stehen unabhängig voneinander ganz besonders bevorzugt für Wasserstoff, Fluor, Chlor, Brom, Methyl, Trifluormethyl, Difluormethyl oder Trichlormethyl
- R¹⁹ und R²⁰: stehen insbesondere bevorzugt jeweils für Wasserstoff.
- R²¹: steht bevorzugt für Wasserstoff, Fluor, Chlor, Brom, Iod, Methyl, Ethyl oder C₁-C₂-Halogenalkyl mit 1 bis 5 Fluor, Chlor und/oder Bromatomen.
- R²¹: steht besonders bevorzugt für Wasserstoff, Fluor, Chlor, Brom, Iod, Methyl, Ethyl, Trifluor-methyl, Difluormethyl, Difluorchlormethyl oder Trichlormethyl.
- R²¹: steht ganz besonders bevorzugt für Wasserstoff, Fluor, Chlor, Brom, Methyl, Trifluormethyl, Difluormethyl oder Trichlormethyl.
- R²¹: steht insbesondere bevorzugt für Methyl.
- R²² und R²³: stehen unabhängig voneinander bevorzugt für Wasserstoff, Fluor, Chlor, Brom, Amino, Nitro, Methyl, Ethyl oder C₁-C₂-Halogenalkyl mit 1 bis 5 Fluor, Chlor und/oder Bromatomen.
- R²² und R²³: stehen unabhängig voneinander besonders bevorzugt für Wasserstoff, Fluor, Chlor, Brom, Nitro, Methyl, Ethyl, Trifluormethyl, Difluormethyl, Difluorchlormethyl oder Trichlormethyl
- R²² und R²³: stehen unabhängig voneinander ganz besonders bevorzugt für Wasserstoff, Fluor, Chlor, Brom, Methyl, Trifluormethyl, Difluormethyl oder Trichlormethyl.
- R²² und R²³: stehen insbesondere bevorzugt jeweils für Wasserstoff.
- R²⁴: steht bevorzugt für Wasserstoff, Fluor, Chlor, Brom, Iod, Methyl, Ethyl oder C₁-C₂-Halogenalkyl mit 1 bis 5 Fluor, Chlor und/oder Bromatomen,
- R²⁴: steht besonders bevorzugt für Wasserstoff, Fluor, Chlor, Brom, Iod, Methyl, Ethyl, Trifluormethyl, Difluormethyl, Difluorchlormethyl oder Trichlormethyl.
- R²⁴: steht ganz besonders bevorzugt für Wasserstoff, Fluor, Chlor, Brom, Methyl, Trifluormethyl, Difluormethyl oder Trichlormethyl.
- R²⁴: steht insbesondere bevorzugt für Methyl.
- R²⁵: steht bevorzugt für Wasserstoff, Fluor, Chlor, Brom, Amino, C₁-C₄-Alkylamino, Di(C₁-C₄-alkyl)amino, Cyano, Methyl, Ethyl oder C₁-C₂-Halogenalkyl mit 1 bis 5 Fluor, Chlor und/oder Bromatomen.
- R²⁵: steht besonders bevorzugt für Wasserstoff, Fluor, Chlor, Brom, Amino, Methylamino, Dimethylamino, Cyano, Methyl, Ethyl, Trifluormethyl, Difluormethyl, Difluorchlormethyl oder Trichlormethyl.
- R²⁵: steht ganz besonders bevorzugt für Wasserstoff, Fluor, Chlor, Brom, Amino, Methylamino, Dimethylamino, Methyl, Trifluormethyl, Difluormethyl oder Trichlormethyl.
- R²⁵: steht insbesondere bevorzugt für Amino, Methylamino, Dimethylamino, Methyl oder Trifluormethyl.
- R²⁶: steht bevorzugt für Fluor, Chlor, Brom, Methyl, Ethyl oder C₁-C₂-Halogenalkyl mit 1 bis 5 Fluor, Chlor und/oder Bromatomen.
- R²⁶: steht besonders bevorzugt für Fluor, Chlor, Brom, Methyl, Ethyl, Trifluormethyl, Difluormethyl, Difluorchlormethyl oder Trichlormethyl.
- R²⁶: steht ganz besonders bevorzugt für Fluor, Chlor, Brom, Methyl, Trifluormethyl, Difluormethyl oder Trichlormethyl.
- R²⁶: steht insbesondere bevorzugt für Methyl, Trifluormethyl oder Difluormethyl.
- R²⁷: steht bevorzugt für Wasserstoff, Fluor, Chlor, Brom, Amino, C₁-C₄-Alkylamino, Di(C₁-C₄-alkyl)amino, Cyano, Methyl, Ethyl oder C₁-C₂-Halogenalkyl mit 1 bis 5 Fluor, Chlor und/oder Bromatomen.
- R²⁷: steht besonders bevorzugt für Wasserstoff, Fluor, Chlor, Brom, Amino, Methylamino, Dimethylamino, Cyano, Methyl, Ethyl, Trifluormethyl, Difluormethyl, Difluorchlormethyl oder Trichlormethyl
- R²⁷: steht ganz besonders bevorzugt für Wasserstoff, Fluor, Chlor, Brom, Amino, Methylamino, Dimethylamino, Methyl, Trifluormethyl, Difluormethyl oder Trichlormethyl.
- R²⁷: steht insbesondere bevorzugt für Amino, Methylamino, Dimethylamino, Methyl oder Trifluormethyl.
- R²⁸: steht bevorzugt für Fluor, Chlor, Brom, Methyl, Ethyl oder C₁-C₂-Halogenalkyl mit 1 bis 5 Fluor, Chlor und/oder Bromatomen.
- R²⁸: steht besonders bevorzugt für Fluor, Chlor, Brom, Methyl, Ethyl, Trifluormethyl, Difluormethyl, Difluorchlormethyl oder Trichlormethyl.
- R²⁸: steht ganz besonders bevorzugt für Fluor, Chlor, Brom, Methyl, Trifluormethyl, Difluormethyl oder Trichlormethyl.
- R²⁸: steht insbesondere bevorzugt für Methyl, Trifluormethyl oder Difluormethyl.
- R²⁹: steht bevorzugt für Fluor, Chlor, Brom, Methyl, Ethyl oder C₁-C₂-Halogenalkyl mit 1 bis 5 Fluor, Chlor und/oder Bromatomen.
- R²⁹: steht besonders bevorzugt für Fluor, Chlor, Brom, Methyl, Ethyl, Trifluormethyl, Difluormethyl, Difluorchlormethyl oder Trichlormethyl.
- R²⁹: steht ganz besonders bevorzugt für Fluor, Chlor, Brom, Methyl, Trifluormethyl, Difluormethyl oder Trichlormethyl.
- R³⁰: steht bevorzugt für Wasserstoff, Methyl oder Ethyl.
- R³⁰: steht besonders bevorzugt für Methyl.
- R³¹: steht bevorzugt für Fluor, Chlor, Brom, Methyl oder Ethyl,
- R³¹: steht besonders bevorzugt für Fluor, Chlor oder Methyl.
- R³²: steht bevorzugt für Methyl, Ethyl oder C₁-C₂-Halogenalkyl mit 1 bis 5 Fluor, Chlor und/oder Bromatomen.
- R³²: steht besonders bevorzugt für Methyl, Ethyl, Trifluormethyl, Difluormethyl, Difluorchlormethyl oder Trichlormethyl.
- R³²: steht ganz besonders bevorzugt für Methyl, Trifluormethyl, Difluormethyl oder Trichlormethyl
- R³²: steht insbesondere bevorzugt für Methyl oder Trifluormethyl.
- R³³: steht bevorzugt für Wasserstoff, Fluor, Chlor, Brom, Methyl, Ethyl oder C₁-C₂-Halogenalkyl mit 1 bis 5 Fluor, Chlor und/oder Bromatomen.
- R³³: steht besonders bevorzugt für Wasserstoff, Fluor, Chlor, Brom, Methyl oder Trifluormethyl
- R³⁴: steht bevorzugt für Fluor, Chlor, Brom, Iod, Hydroxy, C₁-C₄-Alkyl, Methoxy, Ethoxy, Methylthio, Ethylthio, Difluormethylthio, Trifluormethylthio, C₁-C₂-Halogenalkyl oder C₁-C₂-Halogenalkoxy mit jeweils 1 bis 5 Fluor, Chlor und/oder Bromatomen.
- R³⁴: steht besonders bevorzugt für Fluor, Chlor, Brom, Iod, Methyl, Ethyl, n-Propyl, iso-Propyl, n-Butyl, iso-Butyl, sec-Butyl, tert-Butyl, Trifluormethyl, Difluormethyl, Difluorchlormethyl oder Trichlormethyl
- R³⁴: steht ganz besonders bevorzugt für Fluor, Chlor, Brom, Iod, Methyl, Trifluormethyl, Difluormethyl oder Trichlormethyl.
- R³⁵: steht bevorzugt für Wasserstoff, Methyl, Ethyl, C₁-C₂-Halogenalkyl mit 1 bis 5 Fluor-, Chlor- und/oder Bromatomen, C₁-C₂-Alkoxy-C₁-C₂-alkyl, Hydroxymethyl, Hydroxyethyl, Methylsulfonyl oder Dimethylaminosulfonyl
- R³⁵: steht besonders bevorzugt für Wasserstoff, Methyl, Ethyl, Trifluormethyl, Methoxymethyl, Ethoxymethyl, Hydroxymethyl oder Hydroxyethyl.
- R³⁵: steht ganz besonders bevorzugt für Methyl oder Methoxymethyl.
- R³⁶: steht bevorzugt für Wasserstoff, Fluor, Chlor, Brom, Methyl, Ethyl oder C₁-C₂-Halogenalkyl mit 1 bis 5 Fluor-, Chlor- und/oder Bromatomen.
- R³⁶: steht besonders bevorzugt für Wasserstoff, Fluor, Chlor, Brom, Methyl, Ethyl Trifluormethyl, Difluormethyl oder Trichlormethyl.
- R³⁶: steht ganz besonders bevorzugt für Wasserstoff oder Methyl.
- R³⁷: steht bevorzugt für Wasserstoff, Fluor, Chlor, Brom, Iod, Cyano, Methyl, Ethyl, iso-Propyl oder C₁-C₂-Halogenalkyl mit 1 bis 5 Fluor-, Chlor- und/oder Bromatomen.
- R³⁷: steht besonders bevorzugt für Wasserstoff, Fluor, Chlor, Brom, Iod, Cyano, Methyl, Ethyl, iso-Propyl, Trifluormethyl, Difluormethyl, Difluorchlormethyl oder Trichlormethyl.
- R³⁷: steht ganz besonders bevorzugt für Wasserstoff, Methyl, Difluormethyl oder Trifluormethyl
- R³⁸: steht bevorzugt für Wasserstoff, Fluor, Chlor, Brom, Methyl, Ethyl oder C₁-C₂-Halogenalkyl mit 1 bis 5 Fluor, Chlor und/oder Bromatomen.
- R³⁸: steht besonders bevorzugt für Wasserstoff, Fluor, Chlor, Brom, Iod, Methyl oder Trifluormethyl.
- R³⁸: steht ganz besonders bevorzugt für Wasserstoff.
- R³⁹: steht bevorzugt für Methyl, Ethyl, n-Propyl oder iso-Propyl.
- R³⁹: steht besonders bevorzugt Methyl oder Ethyl.

Die oben aufgeführten allgemeinen oder in Vorzugsbereichen aufgeführten Restedefinitionen bzw. Erläuterungen können zwischen den jeweiligen Bereichen und Vorzugsbereichen beliebig kombiniert werden. Sie gelten für die Endprodukte sowie für die Vor- und Zwischenprodukte entsprechend.

Der erste Schritt des erfindungsgemäßen Verfahrens kann in Gegenwart eines Säurebindemittels (einer Base) oder auch ohne Säurebindemittel (Base) durchgeführt werden, wobei entstehende Salzsäure beim Erwärmen entweicht.

Wenn eine Base verwendet wird, kommen bei der Durchführung des ersten Schrittes des erfindungsgemäßen Verfahrens alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie z.B. Natriumhydrid, Natriumamid, Lithiumdiisopropylamid, Natrium-methylat, Natrium-ethylat, Kalium-tert.-butylat, Natrium-tert.-amylat, Kalium-tert.-amylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N-Ethyl-diisopropylamin, N,N-Dimethylanilin, N,N-Dimethyl-benzylamin, Pyridin, Picolin, Ethylmethylpyridin, N-Methylpiperidin, N-Methylmorpholin, N,N-Dimethylaminopyridin, Diazabicyclooctan (DABCO), Diazabicyclononen (DBN) oder Diazabicycloundecen (DBU). Besonders bevorzugt werden Kaliumcarbonat, Natriumhydroxid, Kaliumhydroxid oder Pyridin verwendet.

Als Verdünnungsmittel zur Durchführung des ersten Schrittes des erfindungsgemäßen Verfahrens kommen alle üblichen inerten, organischen Solventien in Frage. Vorzugsweise verwendbar sind gegebenenfalls halogenierte aliphatische, alicyclische oder aromatische Kohlenwasserstoffe, wie Petrolether, Hexan, Heptan, Cyclohexan, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid oder Sulfone, wie Sulfolan; Carbonsäuren, wie Ameisensäure oder Essigsäure. Besonders bevorzugt arbeitet man in Acetonitril, N,N-Dimethylformamid, N,N-Dimethylacetamid, N-Methylpyrrolidon, Toluol, Tetrahydrofuran.

Die Reaktionstemperaturen können bei der Durchführung des ersten Schrittes des erfindungsgemäßen Verfahrens in einem größeren Bereich variiert werden. Im Allgemeinen arbeitet man bei Temperaturen von -10°C bis +150°C.

Bei der Durchführung des ersten Schrittes des erfindungsgemäßen Verfahrens setzt man pro Mol des Acetophenon-Derivats der Formel (III) im Allgemeinen zwischen 0,8 und 1,5 Mol, bevorzugt äquimolare Mengen des Carbonsäurehalogenids der Formel (II) ein.

Die Reaktionszeit kann in Abhängigkeit von der Reaktivität der Edukte bis zu 30 Stunden betragen, wobei der Abbruch der Reaktion bei vollständigem Umsatz auch schon früher erfolgen kann.

Der zweite Schritt des erfindungsgemäßen Verfahrens wird in aliphatischen oder aromatischen Kohlenwasserstoffen wie Petrolether, Hexan, Heptan, Cyclohexan, Methylcyclohexan, Xylol, Toluol, Benzol oder Ethern wie Tetrahydrofuran, Methyl-tert-Butyl-Ether, Diethylether oder in Gemischen der genannten Lösungsmittel, bevorzugt aber in Toluol gegebenenfalls unter Zugabe von Tetrahydrofuran als Verdünnungsmittel durchgeführt.

Die Reaktionstemperaturen können bei der Durchführung des zweiten Schrittes des erfindungsgemäßen Verfahrens in einem größeren Bereich variiert werden. Im Allgemeinen arbeitet man bei Temperaturen von -30°C bis 50°C, vorzugsweise bei Temperaturen von -10°C bis 25°C.

Bei der Durchführung des zweiten Schrittes des erfindungsgemäßen Verfahrens setzt man pro Mol des Acetylanilides der Formel (IV) im Allgemeinen zwischen 1,5 und 3 Mol, bevorzugt 2 bis 2,5 Mol, an Grignard-Reagenz der Formel (V) ein. Ein Teil des Grignard-Reagenz kann auch durch eine Base wie z.B. ein Metallhydrid, ersetzt werden, wobei jedoch mindestens 1 Mol Grignard-Reagenz notwendig sind.

Der zweite Schritt des erfindungsgemäßen Verfahrens wird in der Regel so durchgeführt, dass man als Lösungsmittel bevorzugt Toluol verwendet, welches vorgelegt wird. Dann gibt man beide Edukte [Acetylanilid der Formel (IV) und Grignard-Reagenz der Formel (V)] zum Lösungsmittel hinzu. Dadurch wird ein Umsatz von über 80 % erreicht, während bei üblicher Durchführung einer solchen Reaktion (Verwendung von Tetrahydrofuran, worin das Edukt gelöst ist, mit anschließender Zugabe von Grignard-Reagenz) lediglich 30 % erreicht werden. Die Durchführung bei tieferen Temperaturen verringert unerwünschte Nebenreaktionen, wie z.B. der Austausch von Halogenen im heterocyclischen Teil durch die Grignard-Alkylgruppe.

Die Eliminierung von Wasser erfolgt im dritten Schritt des erfindungsgemäßen Verfahrens unter saurer Katalyse. Man erhält, abhängig von den Bedingungen, das Benzoxazin und isomere Pyrazolyl-alkenylanilide in unterschiedlichen Zusammensetzungen.

Als Verdünnungsmittel zur Durchführung des dritten Schrittes des erfindungsgemäßen Verfahrens kommen alle üblichen inerten, organischen Solventien in Frage. Vorzugsweise verwendbar sind gegebenenfalls halogenierte aliphatische, alicyclische oder aromatische Kohlenwasserstoffe, wie Petrolether, Hexan, Heptan, Cyclohexan, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 2-Methyl-Tetrahydrofuran, 1,2-Dimethoxyethan, 1,2-Diethoxyethan, 1,1-Diethoxymethan, oder Anisol; Alkohole, wie Methanol, Ethanol; Nitrile, wie Acetonitril Propionitril n- oder i-Butyronitril oder Benzonitril; Amide, wie N,N-Dimethylformamid, N,N-Dimethylacetamid, N-Methylformanilid oder N-Methylpyrrolidon; Ester wie Essigsäuremethylester oder Essigsäureethylester, Sulfoxide, wie Dimethylsulfoxid oder Sulfone, wie Sulfolan; Ketone wie Aceton oder Methylisobutylketon; Carbonsäuren, wie Ameisensäure oder Essigsäure; oder Gemische von diesen. Besonders bevorzugt arbeitet man in Toluol, Xylol, Acetonitril, Essigsäureethylester, Tetrahydrofuran, Dioxan, Methanol, Ethanol oder deren Gemische.

Die Zugabe von Wasserfängern wie Orthoestern, Molsieb oder azeotropes Abdestillieren des entstehenden Wassers ist ebenfalls vorteilhaft.

Bei der Durchführung des dritten Schrittes des erfindungsgemäßen Verfahren kommen als Säuren z.B. p-Toluolsulfonsäure, Trifluoressigsäure, Schwefelsäure, Salzsäure, Bromwasserstoffsäure, Phosphorsäure, oder Säurechloride (wie z.B. POCl₃, SOCl₂, COCl₂) infrage.

Man arbeitet bevorzugt bei 60 bis 140°C oder in der Siedehitze des Lösungsmittels mit Reaktionszeiten von 1h bis 48 h, wobei kürzere Reaktionszeiten eher die Bildung des Benzoxazins und längere Reaktionszeiten die Pyrazolylalkenylanilide begünstigen. Orthoester gibt man z.B. in äquimolaren Mengen hinzu, während die Säuren in katalytischen Mengen von 0,1 - 20 Gew.%, bevorzugt 1-10 Gew. % hinzu gegeben werden können.

Das erfindungsgemäße Verfahren wird im Allgemeinen unter leicht erhöhtem Druck durchgeführt. Es ist jedoch auch möglich, abhängig vom verwendeten Katalysator und der Temperatur, unter hohem oder vermindertem Druck - im Allgemeinen zwischen 0,1 bar und 50 bar, bevorzugt zwischen 1 bar und 10 bar - zu arbeiten.

Die Reaktionstemperatur liegt in einem Bereich zwischen 0 und 150°C, bevorzugt zwischen 20 und 100°C.

Die Reaktionszeit ist nicht kritisch und kann in Abhängigkeit von der Ansatzgröße in einem größeren Bereich von 1 h bis 40 h, bevorzugt von 6 h bis 24 h gewählt werden.

Die durch das erfindungsgemäßen Verfahren herstellbaren Alkylanilide der Formel (I) sind wertvolle Fungizide (vgl. WO 03/010149).

Das erfindungsgemäße Herstellen von Alkylaniliden der Formel (I) wird in den nachstehenden Beispielen beschrieben, welche die obige Beschreibung weiter illustrieren. Die Beispiele sind jedoch nicht in einschränkender Weise zu interpretieren.

### Herstellungsbeispiel

### Schritt 1: N-(2-Acetylphenyl)-5-fluor-1,3-dimethyl-1H-pyrazol-4-carboxamid Aus dem Säurechlorid

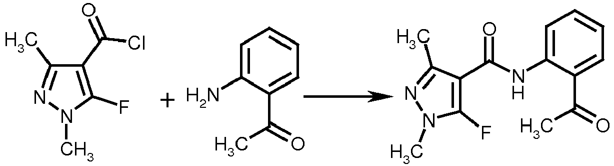

Zu einer Lösung von 2-Aminoacetophenon (45 g, 333,3 mmol) in Toluol (570 ml) tropft man unter Rühren bei 0°C eine Lösung von 1,3-Dimethyl-5-fluor-4-pyrazolcarbonsäurechlorid (58,79 g, 333,3 mmol) in 170 ml Toluol hinzu. Die Lösung wird trübe und man lässt sie auf Raumtemperatur kommen. Dann wird 7 h zum Rückfluss gekocht, wobei unter Aufschäumen HCl-Gas entweicht. Man lässt abkühlen und saugt nach 12 h die ausgefallenen Kristalle ab. Anschließend werden sie in 300 ml Natriumhydrogencarbonatlösung innig gerührt, abgesaugt und mit Wasser gewaschen. Nach Trocknung erhält man 82,6 g (90 % d. Th.) der Zielverbindung N-(2-Acetylphenyl)-5-fluor-1,3-dimethyl-1H-pyrazol-4-carboxamid.

### Aus dem Säurefluorid

Eine Lösung von 2-Aminoacetophenon (405 mg, 3 mmol) und 1,3-Diniethyl-5-fluor-4-pyrazolcarbonsäurefluorid (501 mg, 3 mmol) in Toluol (7 ml) wird 20 h gekocht, wobei nach 16 h nochmals 11 % der Menge an Pyrazolsäurefluorid zugegeben werden. Freiwerdender Fluorwasserstoff wird durch verdünnte Natronlauge geleitet. Man lässt abkühlen und saugt nach 12 h die ausgefallenen Kristalle ab. Anschließend werden sie mit Toluol gewaschen. Nach Trocknung erhält man 598 mg (65 % der Theorie) der Zielverbindung N-(2-Acetylphenyl)-5-fluor-1,3-dimethyl-1H-pyrazol-4-carboxamid in 89%iger Reinheit (GC).

### Schritt 2: 5-Fluor-N-[2-(1-hydroxy-1,3-dimethylbutyl)phenyl]-1,3-dimethyl-1H-pyrazol-4-carboxamid

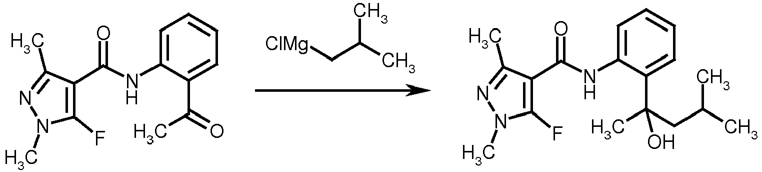

Zu 400 ml Toluol wird unter Argon innerhalb 150 Minuten kontinuierlich unter Rühren bei minus 10°C gleichzeitig N-(2-Acetylphenyl)-5-fluor-1,3-dimethyl-1H-pyrazol-4-carboxamid (als Feststoff, 10 g, 36,3 mmol) und Isobutylmagnesiumchlorid (als 2 M Lösung in THF, 41,7 ml; 83,55 mmol) gegeben. Man lässt auf Raumtemperatur kommen, gibt 200 ml Ammoniumchloridlösung hinzu, extrahiert dreimal mit je 100 ml Essigsäureethylester, trocknet die organischen Phasen mit Natriumsulfat und dampft im Vakuum ein. Man erhält 5-Fluor-N-[2-(1-hydroxy-1,3-dimethylbutyl)phenyl]-1,3-dimethyl-1H-pyrazol-4-carboxamid (14,2 g, Reinheit (GC/MS) 72,8 %; 85% d. Th.), das noch 14 % Ausgangsmaterial enthält.

### Schritt 3: 2-(5-Fluor-1,3-dimethyl-1H-pyrazol-4-yl)-4-isobutyl-4-methyl-4H-3,1-benzoxazin

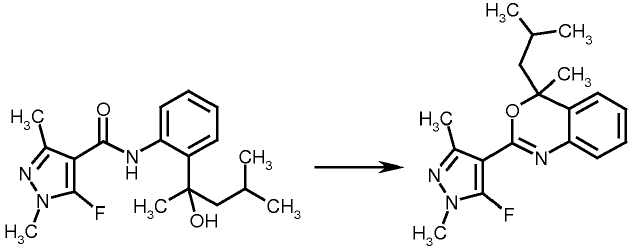

Obiges Produkt aus Stufe 2 wird in 150 ml Toluol mit 0.2 g p-Toluolsulfonsäure 2 h lang am Wasserabscheider gekocht. Man wäscht mit wässriger Natriumhydrogencarbonatlösung und dampft die organische Phase im Vakuum ein. Durch Ausrühren mit Heptan kann das aus der vorherigen Stufe noch enthaltene N-(2-Acetylphenyl)-5-fluor-1,3-dimethyl-1H-pyrazol-4-carboxamid ausgefällt und durch Filtration abgetrennt werden. Nach Chromatographie erhält man 7,2 g (Reinheit 85,1%; 63% der Theorie) von 2-(5-Fluor-1,3-dimethyl-1H-pyrazol-4-yl)-4-isobutyl-4-methyl-4H-3,1-benzoxazin.

### Variante mit Orthoameisensäuretrimethylester

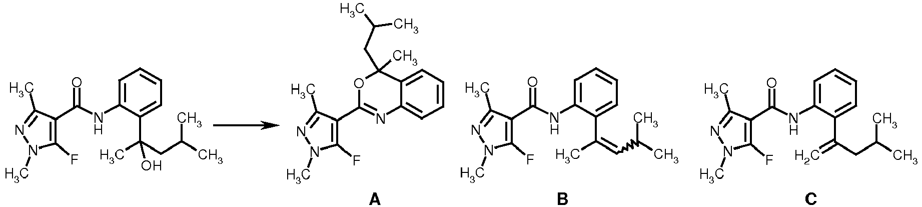

14 g (80%ig, 33,6 mmol) 5-Fluor-N-[2-(1-hydroxy-1,3-dimethylbutyl)phenyl]-1,3-dimethyl-1H-pyrazol-4-carboxamid und 4,28 g (40,3 mmol) Trimethylorthoformiat werden in 420 ml Toluol gelöst und mit 5 Tropfen konzentrierter Schwefelsäure versetzt. Man kocht 1 h, behandelt die Lösung mit Natriumsulfat, filtriert ab und dampft im Vakuum ein. Der Rückstand wird mit 300 ml Heptan behandelt. Dabei fällt N-(2-Acetylphenyl)-5-fluor-1,3-dimethyl-1H-pyrazol-4-carboxamid aus, das noch im Startmaterial enthalten war. Das Filtrat wird einrotiert und man erhält 10,7 g eines gelben Öls. Die GC/MS-Untersuchung zeigt folgende Zusammensetzung: 24 % A, 25 % B, 29 % C sowie 7 % eines Methanol-Addukts.

## Patentansprüche

1. Benzoxazine der Formel 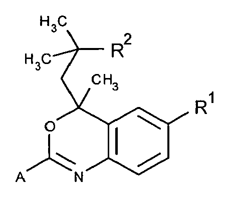 in welcher
R¹ für Wasserstoff oder Fluor steht,
R² für Wasserstoff, Halogen, C₁-C₄-Alkyl oder C₁-C₄-Halogenalkyl mit 1 bis 9 Fluor-, Chlor- und/oder Bromatomen steht,
A für den Rest der Formel (A1) 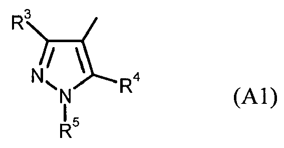 steht, in welcher
R³ für Wasserstoff, Cyano, Halogen, Nitro, C₁-C₄-Alkyl, C₁-C₄-Alkoxy, C₁-C₄-Alkylthio, C₃-C₆-Cycloalkyl, C₁-C₄-Halogenalkyl, C₁-C₄-Halogenalkoxy oder C₁-C₄-Halogenalkylthio mit jeweils 1 bis 5 Halogenatomen, Aminocarbonyl oder Aminocarbonyl-C₁-C₄-alkyl steht,
R⁴ für Wasserstoff, Halogen, Cyano, C₁-C₄-Alkyl, C₁-C₄-Alkoxy oder C₁-C₄-Alkylthio steht,
R⁵ für Wasserstoff, C₁-C₄-Alkyl, Hydroxy-C₁-C₄-alkyl, C₂-C₆-Alkenyl, C₃-C₆-Cycloalkyl, C₁-C₄-Alkylthio-C₁-C₄-alkyl, C₁-C₄-Alkoxy-C₁-C₄-alkyl, C₁-C₄-Halogenalkyl, C₁-C₄-Halogenalkylthio-C₁-C₄-alkyl, C₁-C₄-Halogenalkoxy-C₁-C₄-alkyl mit jeweils 1 bis 5 Halogenatomen, oder Phenyl steht,
oder
A für den Rest der Formel (A2) 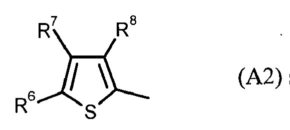 steht, in welcher
R⁶ und R⁷ unabhängig voneinander für Wasserstoff, Halogen, C₁-C₄-Alkyl oder C₁-C₄-Halogenalkyl mit 1 bis 5 Halogenatomen steht,
R⁸ für Halogen, Cyano oder C₁-C₄-Alkyl, oder C₁-C₄-Halogenalkyl oder C₁-C₄-Halogenalkoxy mit jeweils 1 bis 5 Halogenatomen steht,
oder
A für den Rest der Formel (A3) 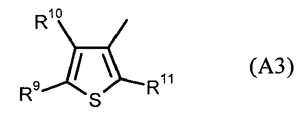 steht, in welcher
R⁹ und R¹⁰ unabhängig voneinander für Wasserstoff, Halogen, C₁-C₄-Alkyl oder C₁-C₄-Halogenalkyl mit 1 bis 5 Halogenatomen steht,
R¹¹ für Wasserstoff, Halogen, C₁-C₄-Alkyl oder C₁-C₄-Halogenalkyl mit 1 bis 5 Halogenatomen steht,
oder
A für den Rest der Formel (A4) 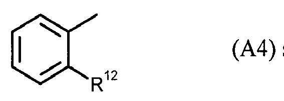 steht, in welcher
R¹² für Wasserstoff, Halogen, Hydroxy, Cyano, C₁-C₆-Alkyl, C₁-C₄-Halogenalkyl, C₁-C₄-Halogenalkoxy oder C₁-C₄-Halogenalkylthio mit jeweils 1 bis 5 Halogenatomen steht,
oder
A für den Rest der Formel (A5) 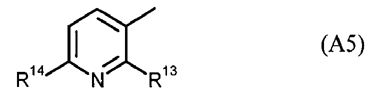 steht, in welcher
R¹³ für Halogen, Hydroxy, Cyano, C₁-C₄-Alkyl, C₁-C₄-Alkoxy, C₁-C₄-Alkylthio, C₁-C₄-Halogenalkyl, C₁-C₄-Halogenalkylthio oder C₁-C₄-Halogenalkoxy mit jeweils 1 bis 5 Halogenatomen steht,
R¹⁴ für Wasserstoff, Halogen, Cyano, C₁-C₄-Alkyl, C₁-C₄-Alkoxy, C₁-C₄-Alkylthio, C₁-C₄-Halogenalkyl, C₁-C₄-Halogenalkoxy mit jeweils 1 bis 5 Halogenatomen, C₁-C₄-Alkylsulphinyl oder C₁-C₄-Alkylsulphonyl steht,
oder
A für den Rest der Formel (A6) 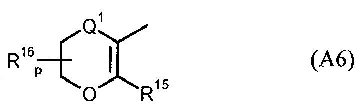 steht, in welcher
R¹⁵ für C₁-C₄-Alkyl oder C₁-C₄-Halogenalkyl mit 1 bis 5 Halogenatomen steht,
R¹⁶ für C₁-C₄-Alkyl steht,
Q¹ für S (Schwefel), O (Sauerstoff), SO, SO₂ oder CH₂ steht,
p für 0, 1 oder 2, wobei R¹⁶ für identische oder verschiedene Reste steht, wenn p für 2 steht,
oder
A für den Rest der Formel (A7) 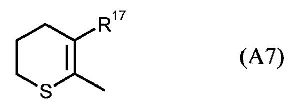 steht, in welcher
R¹⁷ für C₁-C₄-Alkyl oder C₁-C₄-Halogenalkyl mit 1 bis 5 Halogenatomen steht,
oder
A für den Rest der Formel (A8) 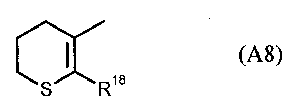 steht, in welcher
R¹⁸ für C₁-C₄-Alkyl oder C₁-C₄-Halogenalkyl mit 1 bis 5 Halogenatomen steht,
oder
A für den Rest der Formel (A9) 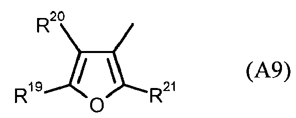 steht, in welcher
R¹⁹ und R²⁰ unabhängig voneinander für Wasserstoff, Halogen, Amino, C₁-C₄-Alkyl oder C₁-C₄-Halogenalkyl mit 1 bis 5 Halogenatomen stehen,
R²¹ für Wasserstoff, Halogen, C₁-C₄-Alkyl oder C₁-C₄-Halogenalkyl mit 1 bis 5 Halogenatomen steht,
oder
A für den Rest der Formel (A10) 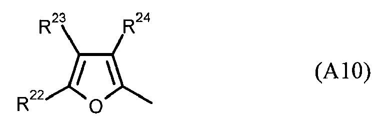 steht, in welcher
R²² und R²³ unabhängig voneinander für Wasserstoff, Halogen, Amino, Nitro, C₁-C₄-Alkyl oder C₁-C₄-Halogenalkyl having 1 bis 5 Halogenatomen stehen,
R²⁴ für Wasserstoff, Halogen, C₁-C₄-Alkyl oder C₁-C₄-Halogenalkyl mit 1 bis 5 Halogenatomen steht,
oder
A für den Rest der Formel (A11) 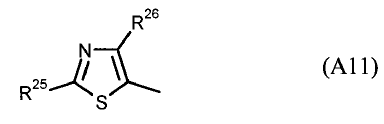 steht, in welcher
R²⁵ für Wasserstoff, Halogen, Amino, C₁-C₄-Alkylamino, Di-(C₁-C₄-alkyl)amino, Cyano, C₁-C₄-Alkyl oder C₁-C₄-Halogenalkyl mit 1 bis 5 Halogenatomen steht,
R²⁶ für Halogen, C₁-C₄-Alkyl oder C₁-C₄-Halogenalkyl mit 1 bis 5 Halogenatomen steht,
oder
A für den Rest der Formel (A12) 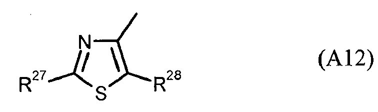 steht, in welcher
R²⁷ für Wasserstoff, Halogen, Amino, C₁-C₄-Alkylamino, Di-(C₁-C₄-alkyl)amino, Cyano, C₁-C₄-Alkyl oder C₁-C₄-Halogenalkyl mit 1 bis 5 Halogenatomen steht,
R²⁸ für Halogen, C₁-C₄-Alkyl oder C₁-C₄-Halogenalkyl mit 1 bis 5 Halogenatomen steht,
oder
A für den Rest der Formel (A13) 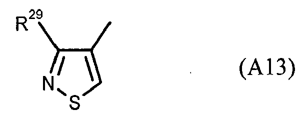 steht, in welcher
R²⁹ für Halogen, C₁-C₄-Alkyl oder C₁-C₄-Halogenalkyl mit 1 bis 5 Halogenatomen steht,
oder
A für den Rest der Formel (A 14) 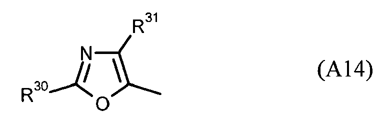 steht, in welcher
R³⁰ für Wasserstoff oder C₁-C₄-Alkyl steht,
R³¹ für Halogen oder C₁-C₄-Alkyl steht,
oder
A für den Rest der Formel (A15) 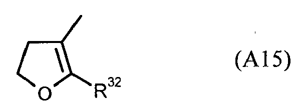 steht, in welcher
R³² für C₁-C₄-Alkyl oder C₁-C₄-Halogenalkyl mit 1 bis 5 Halogenatomen steht,
oder
A für den Rest der Formel (A16) 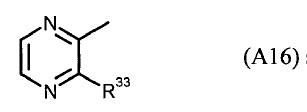 steht, in welcher
R³³ für Wasserstoff, Halogen, C₁-C₄-Alkyl oder C₁-C₄-Halogenalkyl mit 1 bis 5 Halogenatomen steht,
oder
A für den Rest der Formel (A17) 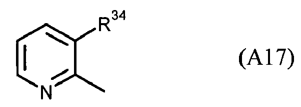 steht, in welcher
R³⁴ für Halogen, Hydroxy, C₁-C₄-Alkyl, C₁-C₄-Alkoxy, C₁-C₄-Alkylthio, C₁-C₄-Halogenalkyl, C₁-C₄-Halogenalkylthio oder C₁-C₄-Halogenalkoxy mit jeweils 1 bis 5 Halogenatomen steht,
oder
A für den Rest der Formel (A18) 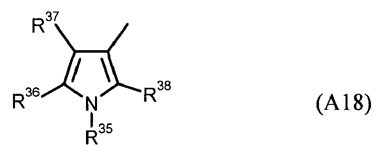 steht, in welcher
R³⁵ für Wasserstoff, Cyano, C₁-C₄-Alkyl, C₁-C₄-Halogenalkyl mit 1 bis 5 Halogenatomen, C₁-C₄-Alkoxy-C₁-C₄-alkyl, Hydroxy-C₁-C₄-alkyl, C₁-C₄-Alkylsulfonyl, Di(C₁-C₄-alkyl)aminosulfonyl, C₁-C₆-Alkylcarbonyl oder jeweils gegebenenfalls substituiertes Phenylsulfonyl oder Benzoyl steht,
R³⁶ für Wasserstoff, Halogen, C₁-C₄-Alkyl oder C₁-C₄-Halogenalkyl mit 1 bis 5 Halogenatomen steht,
R³⁷ für Wasserstoff, Halogen, Cyano, C₁-C₄-Alkyl oder C₁-C₄-Halogenalkyl mit 1 bis 5 Halogenatomen steht,
R³⁸ für Wasserstoff, Halogen, C₁-C₄-Alkyl oder C₁-C₄-Halogenalkyl mit 1 bis 5 Halogenatomen steht,
oder
A für den Rest der Formel (A19) 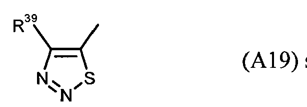 steht, in welcher
R³⁹ für C₁-C₄-Alkyl steht.

## Claims

1. Benzoxazines of the formula 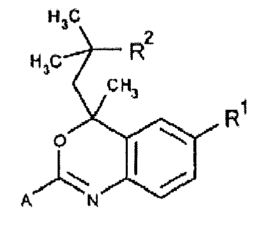 in which
R¹ represents hydrogen or fluorine,
R² represents hydrogen, halogen, C₁-C₄-alkyl or C₁-C₄-haloalkyl having 1 to 9 fluorine, chlorine and/or bromine atoms,
A represents the radical of the formula (A1) 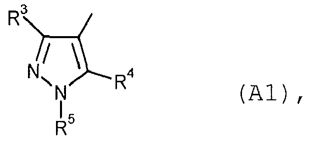 in which
R³ represents hydrogen, cyano, halogen, nitro, C₁-C₄-alkyl, C₁-C₄-alkoxy, C₁-C₄-alkylthio, C₃-C₆-cycloalkyl, C₁-C₄-haloalkyl, C₁-C₄-haloalkoxy or C₁-C₄-haloalkylthio having in each case 1 to 5 halogen atoms, aminocarbonyl or aminocarbonyl-C₁-C₄-alkyl,
R⁴ represents hydrogen, halogen, cyano, C₁-C₄-alkyl, C₁-C₄-alkoxy or C₁-C₄-alkylthio,
R⁵ represents hydrogen, C₁-C₄-alkyl, hydroxy-C₁-C₄-alkyl, C₂-C₆-alkenyl, C₃-C₆-cycloalkyl, C₁-C₄-alkylthio-C₁-C₄-alkylthio-C₁-C₄-alkyl, C₁-C₄-alkoxy-C₁-C₄-alkyl, C₁-C₄-haloalkyl, C₁-C₄-haloalkylthio-C₁-C₄-alkyl, C₁-C₄-haloalkoxy-C₁-C₄-alkyl having in each case 1 to 5 halogen atoms, or phenyl,
or
A represents the radical of the formula (A2) 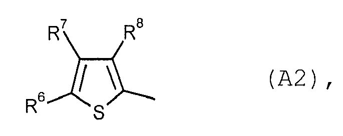 in which
R⁶ and R⁷ independently of one another represent hydrogen, halogen, C₁-C₄-alkyl or C₁-C₄-haloalkyl having 1 to 5 halogen atoms,
R⁸ represents halogen, cyano or C₁-C₄-alkyl, or C₁-C₄-haloalkyl or C₁-C₄-haloalkoxy having in each case 1 to 5 halogen atoms,
or
A represents the radical of the formula (A3) 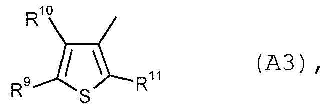 in which
R⁹ and R¹⁰ independently of one another represent hydrogen, halogen, C₁-C₄-alkyl or C₁-C₄-haloalkyl having 1 to 5 halogen atoms,
R¹¹ represents hydrogen, halogen, C₁-C₄-alkyl or C₁-C₄-haloalkyl having 1 to 5 halogen atoms,
or
A represents the radical of the formula (A4) 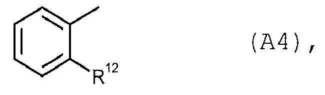 in which
R¹² represents hydrogen, halogen, hydroxyl, cyano, C₁-C₆-alkyl, C₁-C₄-haloalkyl, C₁-C₄-haloalkoxy or C₁-C₄-haloalkylthio having in each case 1 to 5 halogen atoms,
or
A represents the radical of the formula (A5) 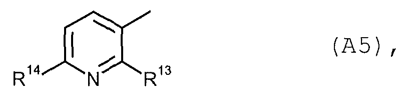 in which
R¹³ represents halogen, hydroxyl, cyano, C₁-C₄-alkyl, C₁-C₄-alkoxy, C₁-C₄-alkylthio, C₁-C₄-haloalkyl, C₁-C₄-haloalkylthio or C₁-C₄-haloalkoxy having in each case 1 to 5 halogen atoms,
R¹⁴ represents hydrogen, halogen, cyano, C₁-C₄-alkyl, C₁-C₄-alkoxy, C₁-C₄-alkylthio, C₁-C₄-haloalkyl, C₁-C₄-haloalkoxy having in each case 1 to 5 halogen atoms, C₁-C₄-alkylsulphinyl or C₁-C₄-alkylsulphonyl,
or
A represents the radical of the formula (A6) 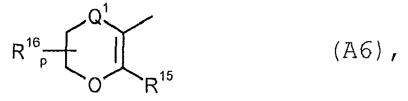 in which
R¹⁵ represents C₁-C₄-alkyl or C₁-C₄-haloalkyl having 1 to 5 halogen atoms,
R¹⁶ represents C₁-C₄-alkyl,
Q¹ represents S (sulphur), 0 (oxygen), SO, SO₂ or CH₂,
p represents 0, 1 or 2, where R¹⁶ represents identical or different radicals if p represents 2,
or
A represents the radical of the formula (A7) 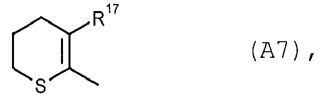 in which
R¹⁷ represents C₁-C₄-alkyl or C₁-C₄-haloalkyl having 1 to 5 halogen atoms,
or
A represents the radical of the formula (A8) 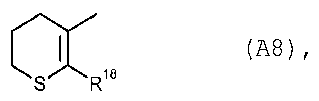 in which
R¹⁸ represents C₁-C₄-alkyl or C₁-C₄-haloalkyl having 1 to 5 halogen atoms,
or
A represents the radical of the formula (A9) 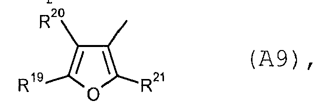 in which
R¹⁹ and R²⁰ independently of one another represent hydrogen, halogen, amino, C₁-C₄-alkyl or C₁-C₄-haloalkyl having 1 to 5 halogen atoms,
R²¹ represents hydrogen, halogen, C₁-C₄-alkyl or C₁-C₄-haloalkyl having 1 to 5 halogen atoms,
or
A represents the radical of the formula (A10) 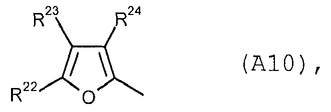 in which
R²² and R²³ independently of one another represent hydrogen, halogen, amino, nitro, C₁-C₄-alkyl or C₁-C₄-haloalkyl having 1 to 5 halogen atoms,
R²⁴ represents hydrogen, halogen, C₁-C₄-alkyl or C₁-C₄-haloalkyl having 1 to 5 halogen atoms,
or
A represents the radical of the formula (A11) 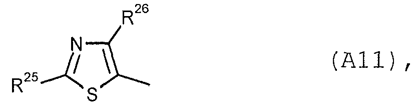 in which
R²⁵ represents hydrogen, halogen, amino, C₁-C₄-alkylamino, di(C₁-C₄-alkyl)amino, cyano, C₁-C₄-alkyl or C₁-C₄-haloalkyl having 1 to 5 halogen atoms,
R²⁶ represents halogen, C₁-C₉-alkyl or C₁-C₄-haloalkyl having 1 to 5 halogen atoms,
or
A represents the radical of the formula (A12) 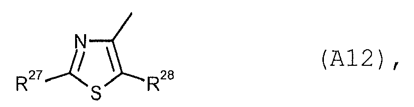 in which
R²⁷ represents hydrogen, halogen, amino, C₁-C₄-alkylamino, di(C₁-C₄-alkyl)amino, cyano, C₁-C₄-alkyl or C₁-C₄-haloalkyl having 1 to 5 halogen atoms,
R²⁸ represents halogen, C₁-C₄-alkyl or C₁-C₄-haloalkyl having 1 to 5 halogen atoms,
or
A represents the radical of the formula (A13) 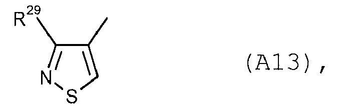 in which
R²⁹ represents halogen, C₁-C₄-alkyl or C₁-C₄-haloalkyl having 1 to 5 halogen atoms,
or
A represents the radical of the formula (A14) 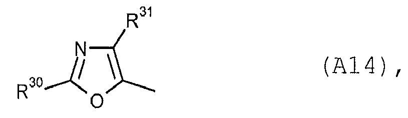 in which
R³⁰ represents hydrogen or C₁-C₄-alkyl,
R³¹ represents halogen or C₁-C₄-alkyl,
or
A represents the radical of the formula (A15) 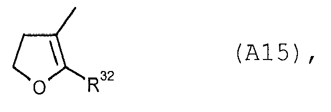 in which
R³² represents C₁-C₄-alkyl or C₁-C₄-haloalkyl having 1 to 5 halogen atoms,
or
A represents the radical of the formula (A16) 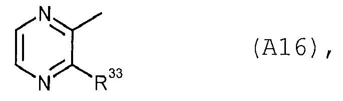 in which
R³³ represents hydrogen, halogen, C₁-C₄-alkyl or C₁-C₄-haloalkyl having 1 to 5 halogen atoms,
or
A represents the radical of the formula (A17) 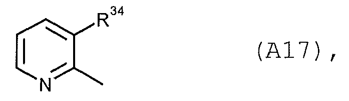 in which
R³⁴ represents halogen, hydroxyl, C₁-C₄-alkyl, C₁-C₄-alkoxy, C₁-C₄-alkylthio, C₁-C₄-haloalkyl, C₁-C₄-haloalkylthio or C₁-C₄-haloalkoxy having in each case 1 to 5 halogen atoms,
or
A represents the radical of the formula (A18) 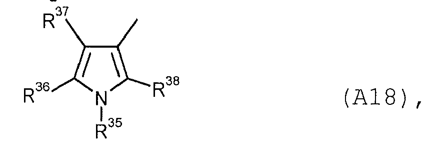 in which
R³⁵ represents hydrogen, cyano, C₁-C₄-alkyl, C₁-C₄-haloalkyl having 1 to 5 halogen atoms, C₁-C₄-alkoxy-C₁-C₄-alkyl, hydroxy-C₁-C₄-alkyl, C₁-C₄-alkylsulphonyl, di(C₁-C₄-alkyl)aminosulphonyl, C₁-C₆-alkylcarbonyl or in each case optionally substituted phenylsulphonyl or benzoyl,
R³⁶ represents hydrogen, halogen, C₁-C₄-alkyl or C₁-C₄-haloalkyl having 1 to 5 halogen atoms,
R³⁷ represents hydrogen, halogen, cyano, C₁-C₄-alkyl or C₁-C₄-haloalkyl having 1 to 5 halogen atoms,
R³⁸ represents hydrogen, halogen, C₁-C₄-alkyl or C₁-C₄-haloalkyl having 1 to 5 halogen atoms,
or
A represents the radical of the formula (A19) 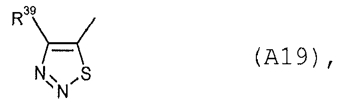 in which
R³⁹ represents C₁-C₄-alkyl.

## Revendications

1. Benzoxazines de formule 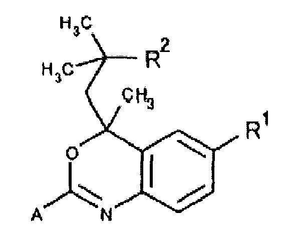 dans laquelle
R¹ représente un atome d'hydrogène ou de fluor,
R² représente un atome d'hydrogène, d'halogène, un groupe alkyle en C₁-C₄ ou halogénoalkyle (C₁-C₄) comportant 1 à 9 atomes de fluor, chlore et/ou brome,
A représente le radical de formule (A1) 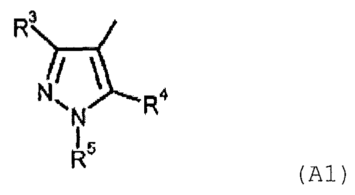 dans laquelle
R³ représente un atome d'hydrogène ou d'halogène, un groupe cyano, nitro, alkyle en C₁-C₄, alcoxy en C₁-C₄, alkyl (C₁-C₄) thio, cycloalkyle en C₃-C₆, halogénoalkyle(C₁-C₄), halogénoalcoxy(C₁-C₄) ou halogénoalkyl(C₁-C₄)-thio comportant chacun de 1 à 5 atomes de carbone, aminocarbonyle ou aminocarbonyl-alkyle(C₁-C₄),
R⁴ représente un atome d'hydrogène, d'halogène, un groupe cyano, alkyle en C₁-C₄, alcoxy en C₁-C₄ ou alkyl(C₁-C₄)thio,
R⁵ représente un atome d'hydrogène, un groupe alkyle en C₁-C₄, hydroxy-alkyle(C₁-C₄), alcényle en C₂-C₆, cycloalkyle en C₃-C₆, alkyl (C₁-C₄)thio-alkyle(C₁-C₄), alcoxy(C₁-C₄)-alkyle(C₁-C₄), halogénoalkyle(C₁-C₄), halogénoalkyl(C₁-C₄)thio-alkyle(C₁-C₄), halogénoalcoxy(C₁-C₄)-alkyle(C₁-C₄) comportant chacun de 1 à 5 atomes d'halogène, ou phényle,
ou
A représente le radical de formule (A2) 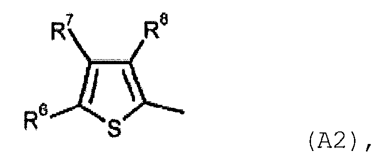 dans laquelle
R⁶ et R⁷ représentent indépendamment l'un de l'autre un atome d'hydrogène, d'halogène, un groupe alkyle en C₁-C₄ ou halogéno-alkyle(C₁-C₄) comportant de 1 à 5 atomes d'halogène,
R⁸ représente un atome d'halogène, un groupe cyano ou alkyle en C₁-C₄, ou halogénoalkyle(C₁-C₄) ou halogénoalcoxy(C₁-C₄) comportant chacun de 1 à 5 atomes d'halogène,
ou
A représente le radical de formule (A3) 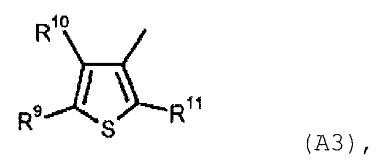 dans laquelle
R⁹ et R¹⁰ représentent indépendamment l'un de l'autre un atome d'hydrogène, d'halogène, un groupe alkyle en C₁-C₄ ou halogéno-alkyle(C₁-C₄) comportant de 1 à 5 atomes d'halogène,
R¹¹ représente un atome d'hydrogène, d'halogène, un groupe alkyle en C₁-C₄, ou halogénoalkyle(C₁-C₄) comportant de 1 à 5 atomes d'halogène,
ou
ou
A représente le radical de formule (A4) 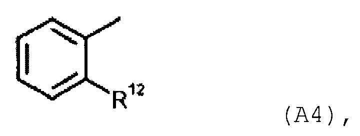 dans laquelle
R¹² représente un atome d'hydrogène, d'halogène, un groupe hydroxy, cyano, alkyle en C₁-C₆, halogénoalkyle(C₁-C₄), halogénoalcoxy(C₁-C₄) ou halogénoalkyl(C₁-C₄)thio comportant chacun de 1 à 5 atomes d'halogène,
ou
A représente le radical de formule (A5) 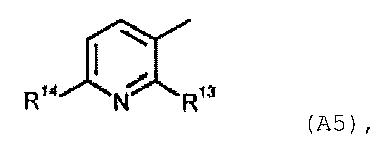 dans laquelle
R¹³ représente un atome d'halogène, un groupe hydroxy, cyano, alkyle en C₁-C₄, alcoxy en C₁-C₄, alkyl(C₁-C₄)thio, halogénoalkyle(C₁-C₄), halogénoalkyl(C₁-C₄)thio ou halogénoalcoxy(C1₋C4₎ comportant chacun de 1 à 5 atomes d'halogène,
R¹⁴ représente un atome d'hydrogène, d'halogène, un groupe cyano, alkyle en C₁-C₄, alcoxy en C₁-C₄, alkyl(C₁-C₄)thio, halogénoalkyle(C₁-C₄), halogénoalcoxy(C₁-C₄) comportant chacun de 1 à 5 atomes d'halogène, alkyl(C₁-C₄)sulfinyle ou alkyl(C₁-C₄)sulfonyle,
ou
A représente le radical de formule (A6) 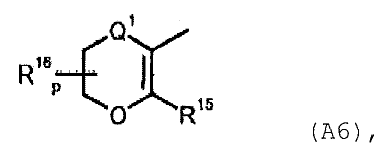 dans laquelle
R¹⁵ représente un groupe alkyle en C₁-C₄ ou halogénoalkyle(C₁-C₄) comportant de 1 à 5 atomes d'halogène,
R¹⁶ représente un groupe alkyle en C₁-C₄,
Q¹ représente S(soufre), 0 (oxygène), SO, SO₂ ou CH₂,
p représente 0, 1 ou 2, R¹⁶ représentant des radicaux identiques ou différents lorsque p représente 2,
ou
A représente le radical de formule (A7) 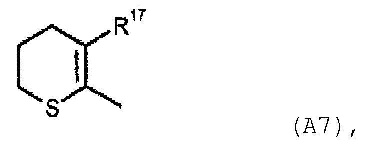 dans laquelle
R¹⁷ représente un groupe alkyle en C₁-C₄ ou halogénoalkyle(C₁-C₄) comportant de 1 à 5 atomes d'halogène,
ou
A représente le radical de formule (A8) 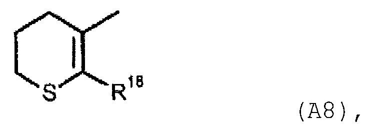 dans laquelle
R¹⁸ représente un groupe alkyle en C₁-C₄ ou halogénoalkyle(C₁-C₄) comportant de 1 à 5 atomes d'halogène,
ou
A représente le radical de formule (A9) 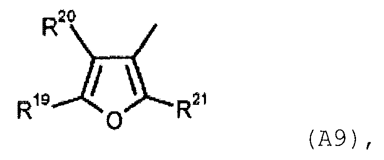 dans laquelle
R¹⁹ et R²⁰ représentent indépendamment l'un de l'autre un atome d'hydrogène, d'halogène, un groupe amino, alkyle en C₁-C₄ ou halogénoalkyle(C₁-C₄) comportant de 1 à 5 atomes d'halogène,
R²¹ représente un atome d'hydrogène, d'halogène, un groupe alkyle en C₁-C₄ ou halogénoalkyle(C₁-C₄) comportant de 1 à 5 atomes d'halogène,
ou
A représente le radical de formule (A10) 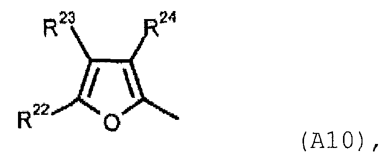 dans laquelle
R²² et R²³ représentent indépendamment l'un de l'autre un atome d'hydrogène, d'halogène, un groupe amino, nitro, alkyle en C₁-C₄ ou halogénoalkyle(C₁-C₄) comportant de 1 à 5 atomes d'halogène,
R²⁴ représente un atome d'hydrogène, d'halogène, un groupe alkyle en C₁-C₄ ou halogénoalkyle(C₁-C₄) comportant de 1 à 5 atomes d'halogène,
ou
A représente le radical de formule (A11) 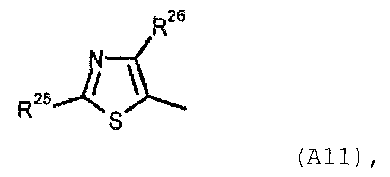 dans laquelle
R²⁵ représente un atome d'hydrogène, d'halogène, un groupe amino, alkyl(C₁-C₄)amino, di(alkyl(C₁-C₄))amino, cyano, alkyle en C₁-C₄ ou halogénoalkyle(C₁-C₄) comportant de 1 à 5 atomes d'halogène,
R²⁶ représente un atome d'halogène, un groupe alkyle en C₁-C₄ ou halogénoalkyle(C₁-C₄) comportant de 1 à 5 atomes d'halogène,
ou
A représente le radical de formule (A12) 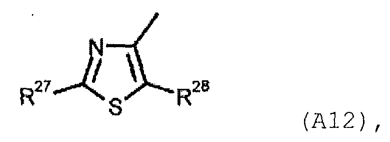 dans laquelle
R²⁷ représente un atome d'hydrogène, d'halogène, un groupe amino, alkyl(C₁-C₄)amino, di(alkyl(C₁-C₄) ) amino, cyano, alkyle en C₁-C₄ ou halogénoalkyle(C₁-C₄) comportant de 1 à 5 atomes d'halogène,
R²⁸ représente un atome d'halogène, un groupe alkyle en C₁-C₄ ou halogénoalkyle (C₁-C_{Q}) comportant de 1 à 5 atomes d'halogène,
ou
A représente le radical de formule (A13) 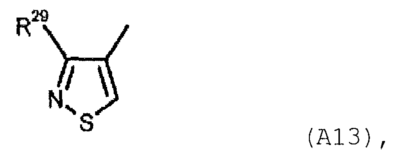 dans laquelle
R²⁹ représente un atome d'halogène, un groupe alkyle en C₁-C₄ ou halogénoalkyle(C₁-C₄) comportant de 1 à 5 atomes d'halogène,
ou
A représente le radical de formule (A14) 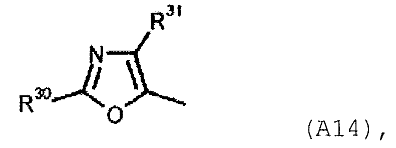 dans laquelle
R³⁰ représente un atome d'hydrogène ou un groupe alkyle en C₁-C₄,
R³¹ représente un atome d'halogène ou un groupe alkyle en C₁-C₄,
ou
A représente le radical de formule (A15) 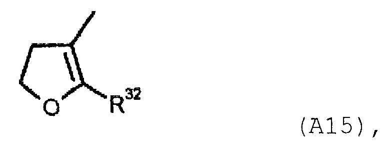 dans laquelle
R³² représente un groupe alkyle en C₁-C₄ ou un groupe halogénoalkyle(C₁-C₄) comportant de 1 à
5 atomes d'halogène,
ou
A représente le radical de formule (A16) 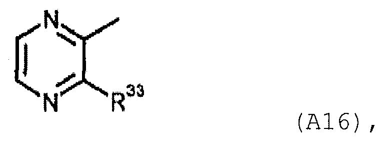 dans laquelle
R³³ représente un atome d'hydrogène, d'halogène, un groupe alkyle en C₁-C₄ ou halogénoalkyle(C₁-C₄) comportant de 1 à 5 atomes d'halogène,
ou
A représente le radical de formule (A17) 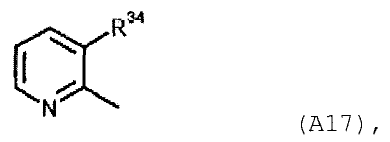 dans laquelle
R³⁴ représente un d'halogène, un groupe hydroxy, alkyle en C₁-C₄, alcoxy en C₁-C₄, alkyl(C₁-C₄)thio, halogénoalkyle(C₁-C₄), halogénoalkyl(C₁-C₄)thio ou halogénoalcoxy(C₁-C₄) comportant chacun de 1 à 5 atomes d'halogène,
ou
A représente le radical de formule (A18) 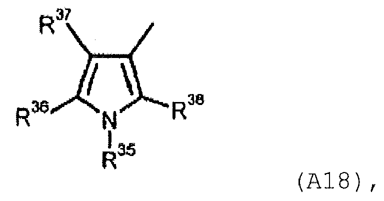 dans laquelle
R³⁵ représente un atome d'hydrogène, un groupe cyano, alkyle en C₁-C₄, halogénoalkyle(C₁-C₄) comportant de 1 à 5 atomes d'halogène, alcoxy(C₁-C₄)-alkyle(C₁-C₄), hydroxyalkyle(C₁-C₄), alkyl(C₁-C₄)sulfonyle, di(alkyl(C₁-C₄)) aminosulfonyle, alkyl(C₁-C₆)-carbonyle ou un groupe phénylsulfonyle ou benzoyle chacun éventuellement substitué,
R³⁶ représente un atome d'hydrogène, d'halogène, un groupe alkyle en C₁-C₄ ou halogénoalkyle(C₁-C₄) comportant de 1 à 5 atomes d'halogène,
R³⁷ représente un atome d'hydrogène, d'halogène, un groupe cyano, alkyle en C₁-C₄ ou halogénoalkyle(C₁-C₄) comportant de 1 à 5 atomes d'halogène,
R³⁸ représente un atome d'hydrogène, d'halogène, un groupe alkyle en C₁-C₄ ou halogénoalkyle(C₁-C₄) comportant de 1 à 5 atomes d'halogène,
ou
A représente le radical de formule (A19) 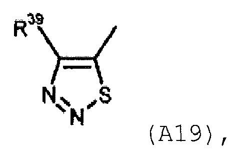 dans laquelle
R³⁹ représente un groupe alkyle en C₁-C₄.
